# EUROPÄISCHE PATENTSCHRIFT

(11) **EP 2 023 863 B1**
(45) Veröffentlichungstag und Bekanntmachung des Hinweises auf die Patenterteilung: **16.03.2016**
(21) Anmeldenummer: 07724591.8
(22) Anmeldetag: 25.04.2007
(51) Int. Cl.: A61F 2/30, A61F 2/40, A61B 17/86

(54) **SCHULTERPROTHESE**
SHOULDER PROSTHESIS
PROTHÈSE D'ÉPAULE

(30) Priorität: 22.05.2006 DE 102006023957; 05.09.2006 DE 102006041551
(43) Veröffentlichungstag der Anmeldung: 18.02.2009
(73) Patentinhaber: Mathys AG Bettlach, 2544 Bettlach (CH)
(72) Erfinder: DALLMANN, Frank, 04626 Schmölln (DE)
(74) Vertreter: Thun, Clemens
(86) Internationale Anmeldenummer: PCT/EP2007/003661
(87) Internationale Veröffentlichungsnummer: WO 2007/134691

(56) Entgegenhaltungen:
- EP-A- 1 520 561
- EP-A- 1 607 070
- EP-A1- 1 064 890
- FR-A1- 2 704 747
- US-A- 4 206 517

## Beschreibung

Die vorliegende Erfindung bezieht sich auf eine Schulterprothese mit einem Gelenkkopf und einer mit diesem zusammenwirkenden Gelenkpfanne. Insbesondere betrifft die vorliegende Erfindung eine sog. inverse Schulterprothese.

Schulterprothesen der gattungsgemäßen Art sind üblicherweise derart aufgebaut, dass eine mit einem Gelenkkopf zusammenwirkende Gelenkpfanne an einem Oberarmknochen, dem sog. Humerus befestigt ist, der mit einem in einer anatomischen Glenoidpfanne (Cavitas Glenoidalis) eines Schulterblatts (Scapula) befestigten Gelenkkopf zusammenwirkt. Im Gegensatz zum anatomischen Schultergelenk, bei dem der kugelartige Humeruskopf des Oberarmknochens mit der Glenoidpfanne des Schulterblatts ein Kugelgelenk bildet, ist die Anordnung zwischen Gelenkkopf und Gelenkpfanne bei dieser Schulterprothese also vertauscht und stellt dementsprechend ein inverses Schultergelenk dar.

Bei diesen inversen Schulterprothesen stellt sich nunmehr das Problem, dass beim Anwinkeln(Adduktion) des Oberarms an den Oberkörper die am Humerus fixierte Gelenkpfanne am Gelenkkopf entlanggleitet, wobei für den Fall, dass der Oberarm sehr stark angewinkelt wird, ein Teilstück der Gelenkpfanne an einem Gelenkkopfrand derart übersteht, dass es an einem Unterrand der Cavitas Glenoidalis des Schulterblatts anstößt. Hierbei kann es zunächst zu Abnutzungserscheinungen und Beschädigungen des Knochenmaterials kommen. Ein weitaus größeres Problem besteht allerdings darin, dass ferner auch die Gefahr besteht, dass der Gelenkpfannenrand zu einem späteren Zeitpunkt mit Schrauben kollidiert, welche zum Verankern des Gelenkkopfs in dem Schulterblatt genutzt werden. Dies kann letztendlich dazu führen, dass diese Schrauben beschädigt werden oder sogar abbrechen, so dass eine sichere Verankerung des Gelenkkopfs in dem Schulterblatt nicht mehr sichergestellt ist.

Um diesen als sog. Inferior Notching bezeichneten Effekt zu vermeiden, wurde bereits versucht, den Gelenkkopf in Richtung einer unteren Ecke des Schulterblatts, dem sog. Angulus Inferior, versetzt an der Cavitas Glenoidalis anzuordnen. Diesbezüglich ist bspw. aus der EP 1 607 070 A1 eine Schulterprothese bekannt, bei der der Gelenkkopf auf eine Grundplatte aufgesteckt ist, die wiederum mit einem zapfartigen Vorsprung am Schulterblatt verankert ist. Der Vorsprung ist in diesem Fall zentrisch zur Mittelachse der Grundplatte schulterblattseitig an dieser angeordnet, wobei der Gelenkkopf eine exzentrisch zu dessen Mittelachse ausgerichtete Aufnahme für die Grundplatte aufweist. Durch die hierbei resultierende versetzte Anordnung des Gelenkkopfs kann zwar der Bewegungsspielraum, bei dem eine Kollision der Gelenkpfanne mit dem Knochenmaterial vermieden wird, erweitert werden, letztendlich wurde allerdings hierdurch das Problem des Inferior Notchings nicht vollständig gelöst. Auch bei dieser Lösung besteht nämlich für den Fall, dass neben dem zapfenartigen Vorsprung zusätzliche Schrauben zur Verankerung der Grundplatte an dem Schulterblatt eingesetzt werden, die Gefahr, dass diese im Laufe der Zeit beschädigt werden.

Eine weitere Problematik der aus der zuvor erwähnten EP 1 607 070 A1 beschriebenen Schulterprothese besteht ferner darin, dass ein zuverlässiges Anordnen des Gelenkkopfs in einer gewünschten Ausrichtung nur schwer an dem Schulterblatt zu realisieren ist. Die übliche Vorgehensweise während der Operation besteht darin, dass ein Arzt zunächst ein dem zapfenartigen Vorsprung entsprechendes Loch in das Schulterblatt einbringt, in welches dann die Grundplatte mit dem Zapfen eingeführt und verankert wird. Hierbei besteht für den Arzt keine Möglichkeit, eine bevorzugte Rotationsorientierung der Grundplatte festzulegen, weshalb durchaus die Gefahr besteht, dass diese verdreht an dem Schulterblatt angeordnet wird. Letztendlich führt dies dazu, dass die Schulterprothese nicht mit der gewünschten Genauigkeit eingesetzt werden kann.

Aus der EP 1 064 890 A1 ist eine Schulterprothese mit einem Gelenkkopf bekannt, der sich über ein Trag- bzw. Stützelement an einem Schulterblatt befestigen lässt. Zur Befestigung des Trag- bzw. Stützelements am Schulterblatt dienen ein am Trag- bzw. Stützelement ausgebildeter geschlitzter Stopfen sowie eine Schraube, die durch ein Durchgangsloch des Trag- bzw. Stützelements geführt ist.

Der vorliegenden Erfindung liegt dementsprechend die Aufgabe zugrunde, die bei der bislang bekannten Schulterprothese bestehenden Nachteile zu vermeiden. Insbesondere soll eine neuartige Schulterprothese mit einer besonders guten und zuverlässigen Implantationstechnik und Kräfteübertragung an den Verankerungsschnittstellen mit dem anatomischen Schulterblatt angegeben werden, um eine erhöhte Materiallebensdauer sowie eine präzisere und verbesserte Verankerungssicherheit zu ermöglichen.

Die Aufgabe wird durch eine Schulterprothese mit den Merkmalen des unabhängigen Anspruchs 1 gelöst. Vorteilhafte Weiterbildungen der Erfindung sind Gegenstand der abhängigen Ansprüche.

Grundsätzlich beruht die vorliegende Erfindung auf der Idee, die Grundplatte, welche zur Befestigung des Gelenkkopfs an dem Schulterblatt vorgesehen ist, mit Hilfe von zwei zapfenartigen Vorsprüngen an der Cavitas Glenoidalis des Scapula zu verankern. Im Gegensatz zu der aus der EP 1 607 070 A1 bekannten Lösung, bei der die Grundplatte mit lediglich einem einzigen Vorsprung befestigt ist, ergeben sich bei der erfindungsgemäßen Lösung deutliche Vorteile hinsichtlich der Vorgehensweise zur Befestigung der Schulterprothese sowie hinsichtlich der hierbei erzielbaren Verankerung. Wie später noch ausführlicher erläutert wird, erlaubt es nämlich der erfindungsgemäße Einsatz zweier Vorsprünge, eine durch das zuvor beschriebene Inferior Notching hervorgerufene Beschädigung zusätzlicher Verankerungselemente zu vermeiden. Des Weiteren erlaubt die Verwendung der beiden Vorsprünge es auch, zusätzlich genutzte Verankerungselemente derart anzuordnen und auszurichten, dass diese in optimaler Weise in das Knochenmaterial des Schulterblatts eingreifen können. Hierdurch wird also eine nochmals verbesserte Verankerung der Schulterprothese am Schulterblatt ermöglicht. Schließlich wird durch die zwei Vorsprünge in Verbindung mit den vorab definierten und eingebrachten Vorbohrungen für die zwei Vorsprünge eine korrekte Rotationsausrichtung der Grundplatte an dem Schulterblatt sichergestellt, weshalb es für einen Arzt beim Anbringen der Schulterprothese deutlich einfacher ist, diese fehlerfrei in der gewünschten Weise zu befestigen.

Erfindungsgemäß wird also eine Schulterprothese mit einer Gelenkpfanne und einem mit dieser zusammenwirkenden Gelenkkopf vorgeschlagen, wobei der Gelenkkopf mit einer scapulaseitigen Grundplatte nach Art einer Klemmverbindung zusammenwirkt, und wobei ferner der Gelenkkopf mit einer in diesem ausgebildeten Aufnahme auf die Grundplatte aufgesteckt ist, welche mit zwei an der Grundplatte angeordneten zapfenartigen Vorsprüngen an der Cavitas Glenoidalis der Scapula verankerbar ist.

Die in den abhängigen Ansprüchen enthaltenen Merkmale betreffen insbesondere die Ausgestaltung der Grundplatte.

So ist zunächst gemäß einer besonders bevorzugten Ausführungsform der vorliegenden Erfindung vorgesehen, dass die Grundplatte in Richtung auf einen Angulus Inferior der Scapula versetzt an der Cavitas Glenoidalis befestigbar ist. Hierzu weist die Grundplatte eine zur Anlage an der Cavitas Glenoidalis des Scapula vorgesehene kalottenförmige Bodenfläche auf, wobei das Zentrum der Kalottenform exzentrisch zur Mittelachse der Grundplatte angeordnet ist. Das Zentrum dieser Kalottenform ist dabei vorzugsweise auf einer Verbindungsgeraden zwischen den beiden Vorsprüngen angeordnet und insbesondere in eine gegenüber dem Angulus Inferior der Scapula entgegengesetzte Richtung verschoben.

Die Aufnahme des Gelenkkopfs für die Grundplatte ist hingegen vorzugsweise zentrisch zu dessen Mittelachse ausgebildet. In Zusammenwirkung mit dem zuvor angesprochenen Merkmal der exzentrischen Ausgestaltung der Bodenfläche der Grundplatte wird hierdurch ermöglicht, dass in einfacher Weise das gewünschte versetzte Anordnen des Gelenkkopfs an dem Schulterblatt erzielt wird. Die Besonderheit besteht hierbei darin, dass die Kontaktfläche der Cavitas Glenoidalis in einfacher Weise kugelabschnitt-förmig ausgestaltet werden kann, um mit der kalottenförmigen Bodenfläche zusammenzupassen. Auch dies trägt zu einer vereinfachten Montage des Gelenkkopfs an dem Schulterblatt bei, da die erforderliche Bearbeitung der Cavitas Glenoidalis durch den Operateur in einfacher Weise durchgeführt werden kann.

Wie bereits zuvor angesprochen wurde, erlaubt die Verwendung der beiden Vorsprünge es insbesondere, zusätzliche Verankerungselemente in vorteilhafter Weise zur Befestigung der Grundplatte an dem Schulterblatt einzusetzen. Gemäß einem weiteren bevorzugten Ausführungsbeispiel ist dementsprechend vorgesehen, dass die Grundplatte zusätzlich mit Verankerungselementen, welche in an der Grundplatte vorgesehenen Bohrungen - hier im Folgenden auch als "Hülsen" bezeichnet - aufnehmbar sind, an der Cavitas Glenoidalis verankerbar ist. Hierbei ist zumindest eine der beiden Hülsen seitlich der Verbindungsgeraden zwischen den beiden Vorsprüngen angeordnet. Vorzugsweise ist zu beiden Seiten der Verbindungsgeraden zwischen den beiden Vorsprüngen jeweils eine Hülse zur Aufnahme eines Verankerungselements angeordnet. Ein besonderer Vorteil dieser Ausgestaltung besteht dabei darin, dass durch die Verwendung der beiden Vorsprünge die beiden Verankerungselemente, bei denen es sich vorzugsweise um Schrauben handelt, mehr zum Zentrum der Grundplatte hin versetzt werden können. Hierdurch ist sichergestellt, dass die Verankerungselemente zuverlässig und sicher in das Knochenmaterial des Schulterblatts eindringen können. Auf diese Weise wird also die Befestigung der Schulterprothese nochmals zusätzlich optimiert.

Die Hülsen können an einer der Scapula abgewandten Seite der Grundplatte Versenkungen für an den Verankerungselementen vorgesehene Köpfe aufweisen. Dabei kann zumindest eine der Hülsen derart ausgebildet sein, dass das entsprechende Verankerungselement nach der endgültigen Montage der Schulterprothese nach Art eines Fingers einer geöffneten Hand weggespreizt ist, wodurch die Verankerung der Prothese in dem Schulterblatt nochmals verbessert wird. Diese Hülse zur winkelstabilen Lagerung eines Verankerungselements weist vorzugsweise ein Schraubgewinde zur Ausbildung einer Schraubverbindung mit dem zugehörigen Verankerungselement auf. Die weiteren Hülsen hingegen sind vorzugsweise derart ausgeführt, dass die zugehörigen Verankerungselemente im wesentlichen parallel zu den Vorsprüngen ausgerichtet sind, wobei allerdings bei der Ausrichtung dieser Verankerungselemente ein geringfügiger Spielraum besteht, um die Montage zu vereinfachen.

Eine Aufnahmeseitenfläche am Gelenkkopf und eine mit dieser zusammenwirkenden Mantelfläche der Grundplatte sind zur Ausbildung der Klemmverbindung vorzugsweise kreisförmig ausgebildet. Um hierbei eine verdrehsichere Anordnung des Gelenkkopfs an der Grundplatte zu ermöglichen, weist der Gelenkkopf an einer dem Angulus Inferior der Scapula abgewandten Seite vorzugsweise eine Nut auf, welche mit einer an der Grundplatte ausgebildeten Feder bzw. Vorwölbung zusammenwirkt. Hierbei kann insbesondere auch vorgesehen sein, dass im Bereich dieser Feder bzw. Vorwölbung an der Grundplatte eine weitere Hülse zur Aufnahme eines zusätzlichen Verankerungselements vorgesehen ist. Die Aufnahme am Gelenkkopf ist dabei vorzugsweise derart dimensioniert, dass die scapulaseitige Bodenfläche der Grundplatte sowie eine scapulaseitige Randfläche des Gelenkkopfs im Wesentlichen bündig ineinander übergehen.

Insgesamt gesehen wird durch diese Ausgestaltungen eine Schulterprothese erhalten, welche in einfacher aber besonders zuverlässiger Weise an dem Schulterblatt angeordnet und verankert werden kann. Darüber hinaus kann eine Beschädigung der zur Verankerung an dem Schulterblatt erforderlichen Elemente weitestgehend ausgeschlossen werden.

Nachfolgend soll die Erfindung anhand der beiliegenden Zeichnungen näher erläutert werden. Es zeigen:
Fig. 1 eine erste Ansicht einer erfindungsgemäßen Schulterprothese mit einer Gelenkpfanne und einem Gelenkkopf sowie mit einer mit dem Gelenkkopf verbundenen Grundplatte mit den entsprechenden Verankerungselementen;
Fig. 2 eine weitere Ansicht der erfindungsgemäßen Schulterprothese;
Fig. 3 und 4 zwei Explosionsdarstellungen der erfindungsgemäßen Schulterprothese;
Fig. 5 eine Ansicht der Grundplatte mit den beiden Vorsprüngen sowie den Verankerungselementen;
Fig. 6 eine Ansicht der Grundplatte ohne Verankerungselemente;
Fig. 7 eine Draufsicht auf die Bodenfläche der Grundplatte;
Fig. 8 und 9 Schnitte durch die Darstellung der Grundplatte gemäß Fig. 7;
Fig. 10 eine Ansicht des mit der Grundplatte verbundenen Gelenkkopfs;
Fig. 11 eine Draufsicht auf die Kombination bestehend aus Grundplatte und Gelenkkopf;
Fig. 12 und 13 zwei Schnitte durch die Darstellung gemäß Fig. 11;
Fig. 14 die Ansicht einer anatomischen Scapula (Schulterblatt);
Fig. 15 eine Schnittdarstellung des an der Scapula befestigten Gelenkkopfs;
Fig. 16 eine weitere Schnittdarstellung der an dem Schulterblatt montierten erfindungsgemäßen Schulterprothese; und
Fig. 17 bis 19 Ansichten einer alternativen Ausführungsform einer erfindungsgemäßen Schulterprothese.

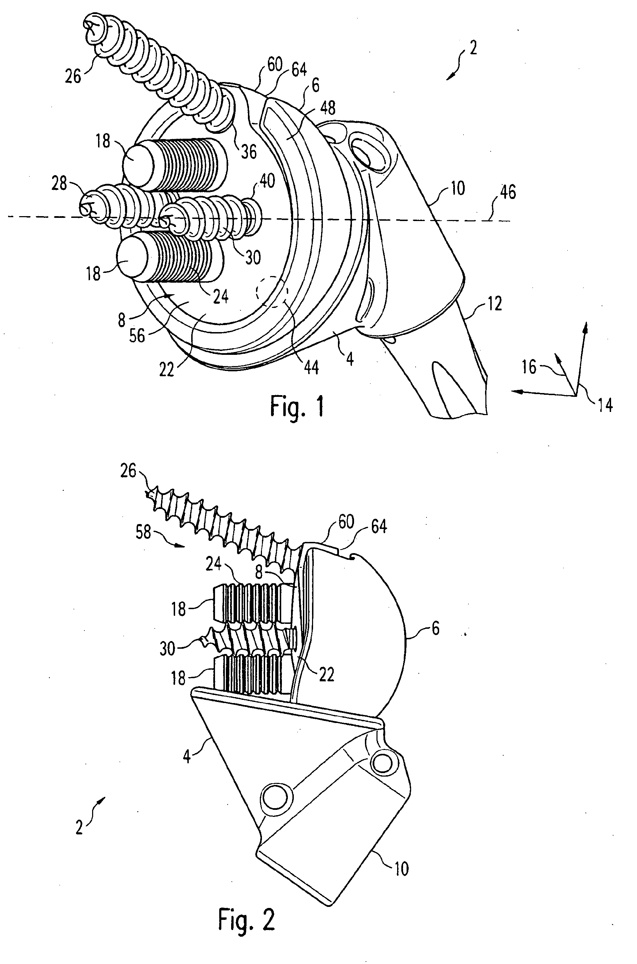

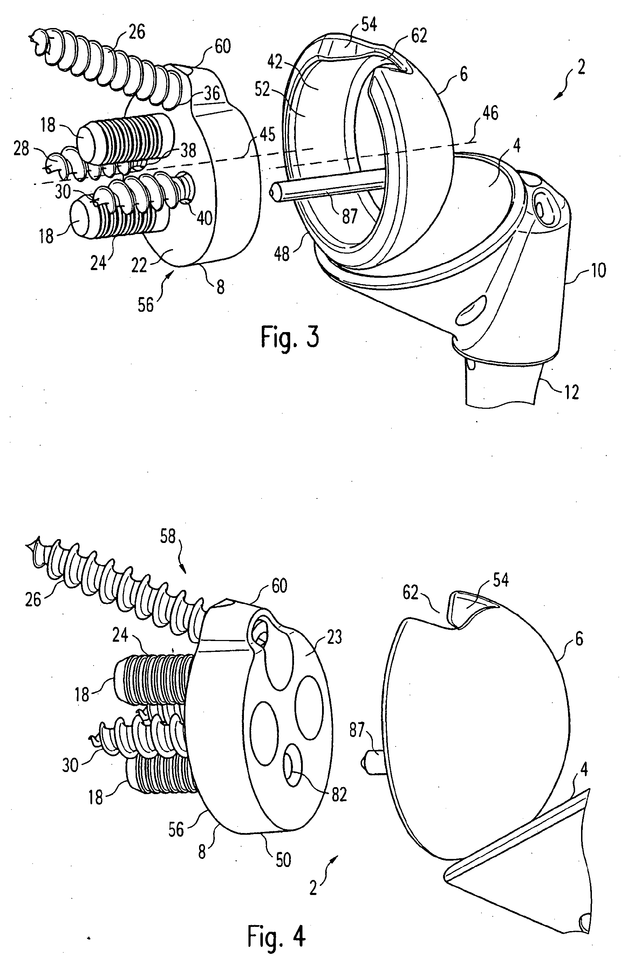

Die Fig. 1 bis 4 zeigen verschiedene Ansichten der allgemein mit dem Bezugszeichen 2 versehenen erfindungsgemäßen Schulterprothese. Fig. 1 und 2 zeigen hierbei die Schulterprothese 2 in zusammengefügtem Zustand, während hingegen die Fig. 3 und 4 Explosionsdarstellungen zeigen.

Wesentliche Bestandteile der Schulterprothese 2 sind eine Gelenkpfanne 4 und ein Gelenkkopf 6, welche nach Art eines Kugelgelenks zusammenwirken. Der Gelenkkopf 6 ist hierbei auf eine Grundplatte 8 aufgesteckt. Die Gelenkpfanne 4 wiederum ist mit Hilfe eines Kopplungsstücks 10 mit einem Stab 12 verbunden, welcher über einen in den Fig. 1 bis 4 nicht dargestellten Humeruskopf im Oberarm (Humerus) versenkt ist. Die Gelenkpfanne 4 ist in und entgegen den mit den Richtungspfeilen 14 und 16 gekennzeichneten Richtungen auf der Oberfläche des Gelenkkopfs 6, der sog. Glenosphäre schwenkbar angeordnet. Fig. 1 zeigt hierbei die Gelenkpfanne 4 in einer Position, bei welcher der Oberarm angewinkelt ist, allerdings nicht derart stark angewinkelt, dass eine Beschädigung des Knochenmaterials zu befürchten wäre.

Zur Verankerung der erfindungsgemäßen Schulterprothese 2 an einem in den Fig. 1 bis 4 nicht gezeigten Schulterblatt, der sog. Scapula, weist die Grundplatte 8 unterschiedliche Elemente auf, welche in das entsprechende Knochenmaterial des Schulterblatts eingebracht werde.

Es handelt sich hierbei zunächst um zwei zapfenartige und einstückig mit der Grundplatte 8 ausgebildete Vorsprünge 18, welche im wesentlichen rechtwinklig zur Bodenfläche 22 der Grundplatte 8 angeordnet sind. Diese Zapfen 18 sind nach Art eines Dübels ausgebildet und weisen an ihrem Umfang Rippen 24 auf. Bei der Montage der Schulterprothese 2 am Schulterblatt werden diese Zapfen 18 in operativ eingebrachte Öffnungen versenkt und mittels Presspassung verklemmt.

Weitere Verankerungselemente stellen drei Schrauben 26, 28 und 30 dar, welchen in an der Grundplatte 8 ausgebildeten Hülsen 36, 38 bzw. 40 aufgenommen sind. Die Verankerungsschrauben werden nach dem Verankern der Grundplatte 8 mit Hilfe der beiden Vorsprünge 18 in das Knochenmaterial des Schulterblatts eingeschraubt, um für eine zusätzliche Verankerung zu sorgen. Sie sind dabei derart an der Grundplatte 8 geführt, dass die Schraube 26 leicht zur Oberseite hin gespreizt ist und die beiden weiteren Schrauben 28 und 30 im wesentlichen parallel zu den Vorsprüngen 18 ausgerichtet sind. Diese beiden Schrauben 28 und 30 dienen primär dazu, die Grundplatte 8 anfänglich an dem Schulterblatt festzuschrauben. Anschließend wird die winkelstabil geführte Schraube 26 eingebracht und hierbei die Grundplatte 8 mit dem Knochen verspreizt, wodurch eine besonders zuverlässige Verankerung der Grundplatte 8 und damit der gesamten Prothese 2 an dem Schulterblatt bewirkt wird.

Der Gelenkkopf 6 umfasst zum Zusammenfügen mit der Grundplatte 8 eine Aufnahme 42 (siehe Fig. 3), deren Ausmaß zur Ausbildung einer Klemmverbindung 44 zwischen dem Gelenkkopf 6 und der Grundplatte 8. Die Aufnahme 42 ist hierbei zentrisch zur Mittelachse 46 des Gelenkkopfs 6 an diesem ausgebildet. Zur Ausbildung der Klemmverbindung 44 zwischen dem Gelenkkopf 6 und der Grundplatte 8 wirkt dementsprechend eine Mantelfläche 50 der Grundplatte 8 mit einer Aufnahmeseitenfläche 52 zusammen. Die Mantelfläche 50 und die Aufnahmeseitenfläche 52 sind hierbei kreisförmig derart ausgebildet, dass eine durch die Randfläche 48 begrenzte Gelenkkopfwand 54 im Bereich der Randfläche 48 der Glenosphäre eine gleichbleibende Wandstärke aufweist. Diese ist das Ergebnis der zentrischen Aufnahme für die Grundplatte 8 und hat zur Folge, dass eine besonders gleichmäßige Kraftübertragung zwischen Grundplatte 8 und Gelenkkopf 6 sichergestellt ist. Im diesem Zusammenhang ist anzumerken, dass die Verbindung zwischen Grundplatte 8 und Gelenkkopf 6 auch anderweitig durch form- und/oder kraftschlüssig zusammenwirkende Elemente realisiert werden könnte. Ein weiteres Beispiels hierfür wird später noch erläutert. Zur Herstellung der beiden Elemente können dabei sämtliche in der Prothetik gängigen Materialien eingesetzt werden, wobei durchaus auch die Möglichkeit einer Paarung von Elementen aus verschiedenen Materialien besteht.

Wie später noch näher erläutert wird, weist die Bodenfläche 22 der Grundplatte 8 eine leichte Kalottenform auf, wobei das Zentrum dieser Kalottenform gegenüber der Mittelachse 45 der Grundplatte 8 und damit auch gegenüber der Mittelachse des Gelenkkopfs 46 versetzt ist. Hieraus ergeben sich besondere Vorteile hinsichtlich der Montage der Schulterprothese 2 am Schulterblatt, welche ebenfalls später noch näher erläutert werden.

Zu bemerken ist ferner, dass die Aufnahme 42 des Gelenkkopfs 6 derart dimensioniert ist, dass die Grundplatte 8 beim Aufstecken des Gelenkkopfs 6 auf die Grundplatte 8 nahezu vollständig in der Aufnahme 42 verschwindet. Insbesondere schließt die Randfläche 48 des Gelenkkopfs 6 nahezu bündig an die Bodenfläche 22 der Grundplatte 8 an.

Um eine Beschädigung des Knochenmaterials des Schulterblatts beim starken Anwinkeln des Arms möglichst zu vermeiden, sollte die Grundplatte 8 auf der in den Fig. 1 bis 4 nicht dargestellten Cavitas Glenoidalis entgegen der Pfeilrichtung 14 versetzt fixiert werden. Dies bedeutet, dass die Grundplatte 8 mit einem unteren Teilstück 56 nicht mit der Bodenfläche 22 auf der Cavitas Glenoidalis aufliegen kann. Zu diesem Zweck ist eine Erhöhung der Verankerungsstabilität der Grundplatte 8 wünschenswert, was dadurch erzielt wird, dass die Hülse 36 zur Aufnahme einer der Verankerungsschrauben 26 in einem oberen Teilstück 58 der Grundplatte 8 ausgebildet ist. Um diese Hülse 36 am oberen Teilstück 58 der Grundplatte ausbrechsicher an dieser auszubilden, weist diese eine Vorwölbung bzw. eine Feder 60 auf, welche zunächst den notwendigen Platz für die Hülse 36 zur Verfügung stellt. Des Weiteren wirkt diese Feder 60 mit einer am Gelenkkopf vorgesehen Nut 62 zusammen, um eine Nut-Feder-Verbindung 64 zu bilden, welche ein fehlerfreies Aufstecken des Gelenkkopfs 6 auf die Grundplatte 8 ermöglicht. Durch diese Nut-Feder-Verbindung 64 wird ferner auch ein Verdrehen des Gelenkkopfs 6 auf der Grundplatte 8 durch auf den Gelenkkopf 6 radial einwirkende Kräfte vermieden.

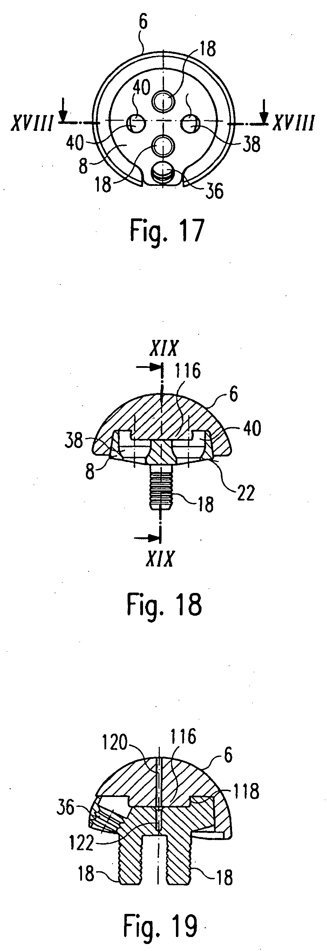

Ein weiteres Element, welches zum lagerichtigen Aufstecken des Gelenkkopfs 6 auf die Grundplatte 8 dient, ist ein von der Innenseite des Gelenkkopfs 6 vorstehender Zentrierstift 87, der in eine Öffnung 82 der Grundplatte 8 eingreift. Diese Öffnung 82 an der Grundplatte 8 erstreckt sich dabei bis in den unteren Vorsprung 18, so dass eine ausreichende Länge zur Aufnahme des Zentrierstift 87 zur Verfügung steht. Es handelt sich hierbei um optionale Hilfsmittel, welche das Aufsetzen des Gelenkkopfs 6 auf die an dem Knochen befestigte Grundplatte 8 erleichtern. Gegebenenfalls könnte auf diese Hilfsmittel auch verzichtet werden bzw. es wäre die Verwendung alternativer Hilfsmittel denkbar. Eine mögliche Variante ist beispielsweise in den Figuren 17 bis 19 gezeigt.

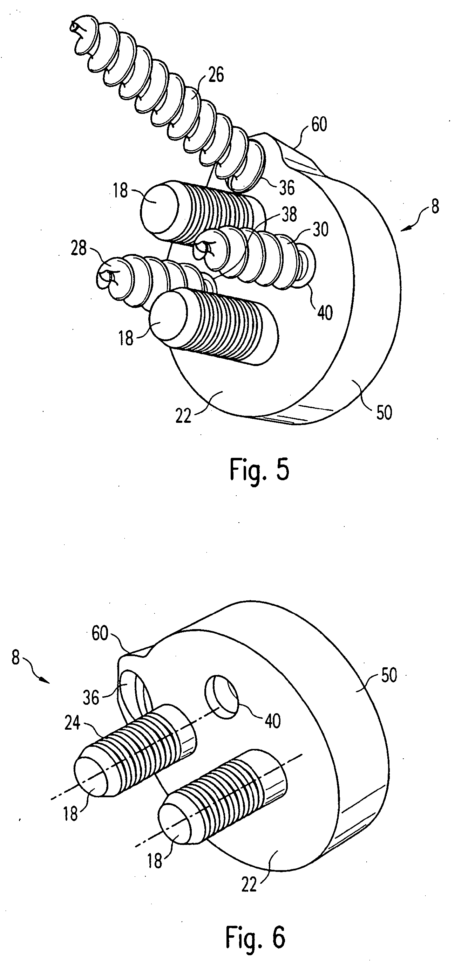

Fig. 5 zeigt eine Draufsicht auf die Grundplatte 8 in einem Zustand, in dem auch die Verankerungsschrauben 26, 28, 30 in die entsprechenden Hülsen 36, 38, 40 eingebracht wurden. Die entsprechenden Öffnungen zum Einführen der Schrauben 26, 28, 30 können hierbei der Explosionsdarstellung von Fig. 4 entnommen werden, in der die Rückseite 23 der Grundplatte 8 gezeigt ist. Gezeigt ist neben den drei Öffnungen für die Verankerungsschrauben 26, 28, 30 auch die Öffnung 82 zur Aufnahme des Zentrierstifts 87.

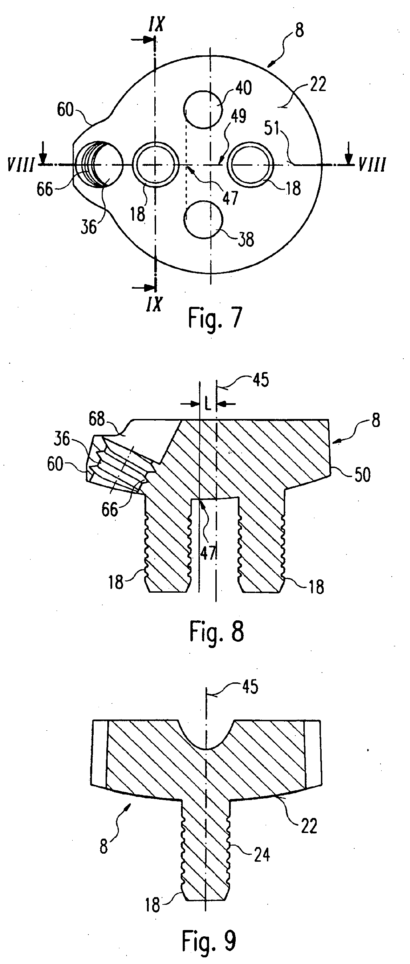

Anhand der Fig. 6 bis 9 soll im folgenden der Aufbau der Grundplatte 8 näher beschrieben werden. Hierbei ist insbesondere von Bedeutung, dass die Bodenfläche 22 der Grundplatte 8 die zuvor angesprochene Kalottenform aufweist. Ferner ist in diesem Zusammenhang hervorzuheben, dass das Zentrum 47 der Kalottenform nicht mit dem geometrischen Zentrum bzw. der Mittelachse 45 der im wesentlichen kreisförmigen Grundplatte 8 zusammenfällt. Stattdessen ist das Zentrum 47 der Kalottenform um eine Distanz L leicht in Richtung auf die Hülse 36 für die obere Verankerungsschraube 26 hin verschoben. In dieser Richtung weist also die Bodenfläche 22 der Grundplatte 8 eine gewisse Exzentrität auf. Das Zentrum 47 der Kalottenform liegt hierbei auf der Verbindungsgeraden 49 zwischen den beiden Vorsprüngen 18 bzw. auf der durch die Vorsprünge 18 und die Feder 60 verlaufenden Symmetrieachse 51, wie einerseits der Darstellung von Fig. 7 und andererseits der Schnittdarstellung von Fig. 9 entnommen werden kann.

Der Darstellung von Fig. 7 kann ferner auch entnommen werden, dass die Hülsen 38 und 40 für die beiden weiteren Verankerungsschrauben 28 und 30 symmetrisch zu der Symmetrieachse 51 angeordnet sind. Insbesondere befinden sich die Hülsen 38, 40 zu beiden Seiten der Symmetrieachse 47 jeweils im Bereich zwischen den beiden Vorsprüngen 18. Da keiner der beiden Vorsprünge 18 unmittelbar im Zentrum der Grundplatte 8 ausgebildet ist, besteht dementsprechend die Möglichkeit, die Aufnahmehülsen 38, 40 näher zur Symmetrieachse 51 zu verlegen, was - wie nachfolgend noch näher erläutert wird - besondere Vorteile beim Verankern der Grundplatte 8 an dem Knochenmaterial des Schulterblatts mit sich bringt.

Hervorzuheben ist ferner, dass die Hülse 36, welche wie zuvor erwähnt zur winkelstabilen Lagerung der Schraube 26 vorgesehen ist, ein Schraubgewinde 66 aufweist. Mit Hilfe dieses Gewindes 66 kann die Schraube 26, welche zum Verspreizen des Grundplatte 8 mit dem Knochen vorgesehen ist, über eine Schraubverbindung mit der Hülse 36 zusammenwirken. Die Hülse 36 ist hierzu ferner auch mit einer Versenkung 68 zur Aufnahme des entsprechenden Schraubenkopfes versehen.

Die beiden weiteren Hülsen 38, 40 hingegen sind anders ausgestaltet als die Hülse 36, da diese zur Halterung der Schrauben 28 und 30 vorgesehen sind, welche eine andere Funktion erfüllen. Diese Schrauben 28 und 30 dienen primär dazu, die Grundplatte 8 an dem Schulterblatt zunächst festzuschrauben, bevor mit Hilfe der Schraube 26 die Spreizverbindung hervorgerufen wird. Die Hülsen 38 und 40 sind deshalb im wesentlichen parallel zu den Vorsprüngen 18 ausgerichtet, weisen keine Gewinde auf und sind ferner in ihrem Kopfbereich kugelabschnitt-förmig ausgebildet. Werden als Verankerungselemente Schrauben mit einem kugelig ausgebildeten Kopf verwendet, so können diese in einem kleinen Winkelbreich geschwenkt werden. Dieser geringfügige Spielraum ermöglicht ein einfacheres Einbringen der Schrauben in das Schulterblatt, wodurch die Montage der Prothese erleichtert wird.

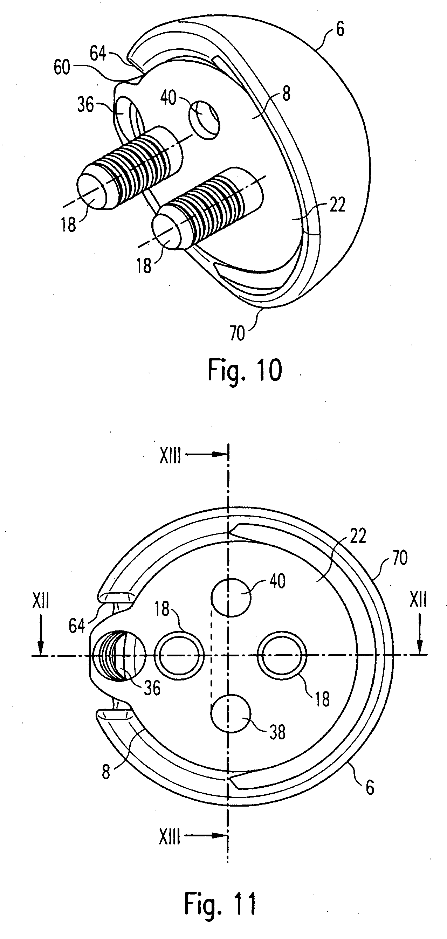

Diese Ausgestaltung der Hülsen 38 und 40 kann insbesondere auch den Fig. 10 bis 13 entnommen werden, welche das Zusammenwirken der Grundplatte 8 mit dem Gelenkkopf 6 zeigen und nachfolgen erläutert werden sollen.

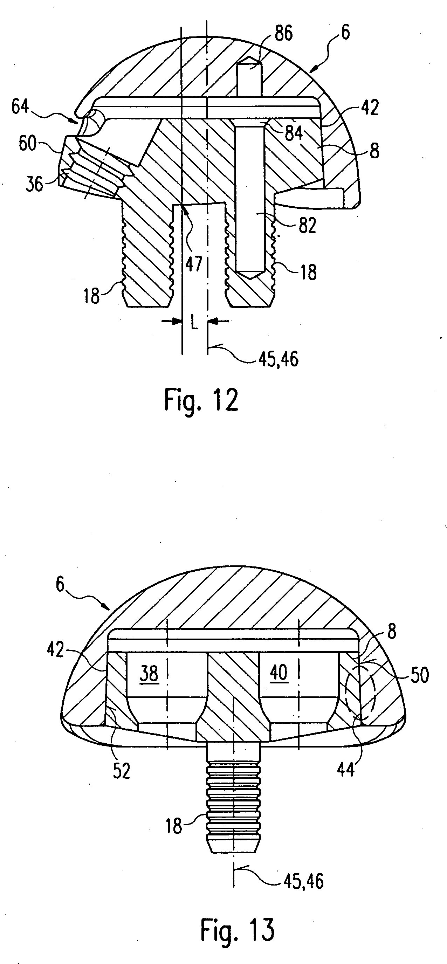

Der Gelenkkopf umfasst dementsprechend die Aufnahme 42, welche - wie insbesondere der Darstellung von Fig. 13 entnommen werden kann - zentrisch zu dessen Mittelachse 46 an diesem ausgebildet ist. Mit Ausnahme der Nut-Feder-Verbindung 64 weist dementsprechend die Umfangswand des Gelenkkopfs 6 eine gleichmäßige Dicke auf, wodurch eine besonders sichere Halterung des Gelenkkopfs 6 auf der Grundplatte 8 ermöglicht ist. Hierzu trägt die Klemmverbindung 44 zwischen der Aufnahmeseitenfläche 52 des Gelenkkopfs 6 und der Mantelfläche 50 der Grundplatte 8 bei auf die Grundplatte 8 aufgesteckten Gelenkkopf 6 bei.

Insbesondere der Darstellung in Fig. 12 kann dabei nochmals die exzentrische Ausgestaltung der Bodenfläche 22 in der Grundplatte 8 entnommen werden. Deutlich ist erkennbar, dass das Zentrum 47 der vorgewölbten Bodenfläche 22 gegenüber der Mittelachse 45 der Grundplatte 8 leicht in Längsrichtung auf die Nut-Feder-Verbindung 64 hin verschoben ist. Eine gegenüber der Symmetrieachse seitliche Abweichung hingegen liegt gemäß der Darstellung von Fig. 13 nicht vor, d.h., das Zentrum der Kalottenform fällt in dieser Richtung mit den Mittelachsen 45 und 46 der Grundplatte 8 und des Gelenkkopfs 6 zusammen.

Der Darstellung von Fig. 12 ist ferner noch die Bohrung 82 der Grundplatte 8 entnehmbar, welche gelenkkopfseitig ein offenes Ende 84 aufweist und scapulaseitig bis in den entsprechenden Vorsprung 18 hineinreicht. In Verlängerung dieser Bohrung 82 ist auch im Gelenkkopf 6 eine entsprechende Ausnehmung 86 ausgebildet, welche zur Aufnahme des in den Fig. 1 bis 4 dargestellten Zentrierstifts 87 zur Zentrierung des Gelenkkopfs 6 beim Aufstecken auf die Grundplatte 8 dient. Gemeinsam mit der Nut-Feder-Verbindung 64 wird hierdurch ein verdrehsicheres Anordnen des Gelenkkopfs 6 an der Grundplatte 8 ermöglicht sowie das Aufsetzen des Gelenkkopfes 6 auf die vormontierte Grundplatte 8 erleichtert.

Eine weitere Besonderheit, die insbesondere den Fig. 10 bis 12 entnommen werden kann, ist, dass die Mantelfläche des Gelenkkopfs 6 in einem der Nut-Feder-Verbindung 64 gegenüberliegenden Bereich leicht nach unten gezogen ist. Diese Verlängerung der Mantelfläche in Form einer Lippe 70 hat zur Folge, dass beim Anwinkeln des Oberarms eine zusätzliche bzw. verlängerte Führung für die Gelenkpfanne 4 geschaffen wird. Ein weiterer Vorteil dieser Ausgestaltung besteht darin, dass die Kontaktfläche zwischen der Oberfläche des Gelenkkopfs 6 und der Gelenkpfanne 4 beim Abwinkeln des Arms vergrößert wird, so dass letztendlich eine gleichmäßigere Kraftübertragung stattfinden kann. Insbesondere wird das Entstehen von Punkt- oder Linienkontakten, welche zu einer verstärkten Abnutzung des Materials führen würden, reduziert. In diesem Zusammenhang ist auch zu erwähnen, dass der Gelenkkopf 6 über seine gesamte Außenseite hinweg eine geschlossene Oberfläche bildet. Insbesondere sind keine Öffnungen oder dergleichen vorhanden, welche möglicherweise zu Punkt- oder Linienkontakten mit der Gelenkpfanne 4 führen könnten, was im Hinblick auf eine evtl. Abnutzung des Materials deutliche Vorteile mit sich bringt.

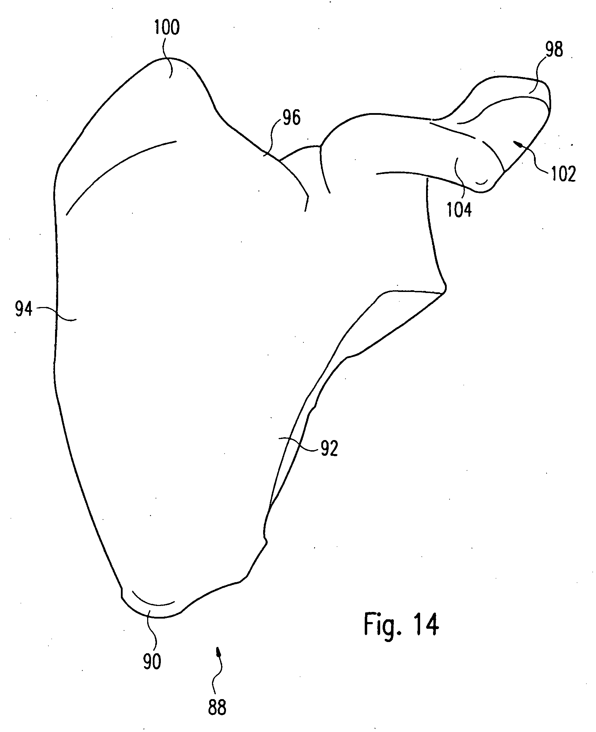

Fig. 14 zeigt eine Draufsicht einer anatomischen Scapula 88, dem Schulterblatt, deren Grundform ein Dreieck darstellt. Dabei wird das untere Eck des Dreiecks als Angulus Inferior 90 bezeichnet, woran ein rechter Rand "Margo Lateralis 92" und ein linker Rand "Margo Medialis 94" anschließen. Der Margo Lateralis 92 und der Margo Medialis 94 begrenzen den oberen Rand "Margo Superior 96" an einem rechten Eck "Angulus Lateralis 98" bzw. an einem linken Eck "Angulus Superior 100". Am Angulus Lateralis 98 ist eine anatomische Gelenkpfanne "Cavitas Glenoidalis 102" positioniert, welche einen nach vorne und außen gekrümmten Rabenschnabelfortsatz "Processus Coracoideus 104" aufweist.

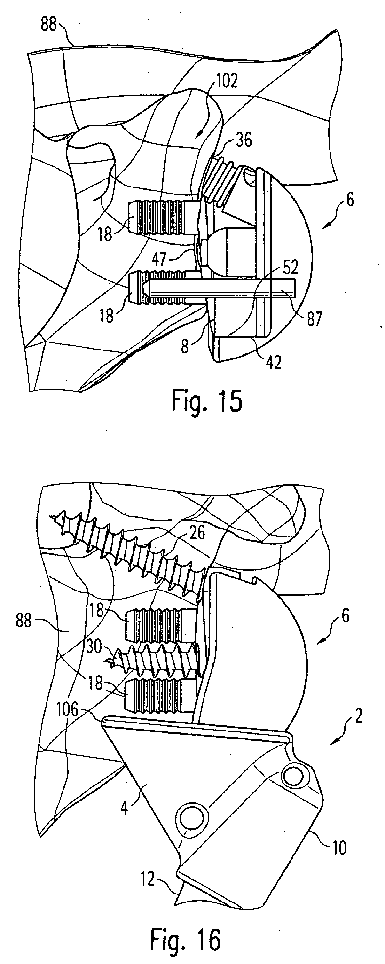

Die Fig. 15 und 16 zeigen nunmehr den am Schulterblatt mit Hilfe der Grundplatte 8 befestigten Gelenkkopf 6 bzw. die vollständig an dem Schultergelenk befestigte Schulterprothese 2. Die über das Kopplungsstück 10 am Stab 12 befestigte Gelenkpfanne 4 ist hierbei am nicht gezeigten Oberarmknochen (Humerus) fixiert. Die Grundplatte 8 wiederum ist an der Cavitas Glenoidalis 102 der Scapula 88 mit den Vorsprüngen 18 sowie den Verankerungsschrauben 26, 28 und 30 verankert. Der Gelenkkopf 6 ist mit der Aufnahme 42 auf die Grundplatte 8 aufgesteckt. Die Aufnahme 42 ist zentrisch um die Mittelachse 46 des Gelenkkopfs 6 an diesem ausgebildet und seitlich von einer Aufnahmeseitenfläche 52 begrenzt, welche kreisförmig um die Mittelachse 46 verläuft.

Die Grundplatte 8 wiederum ist mit den Vorsprüngen 18 und den Verankerungsschrauben 26, 28, 30 an der Cavitas Glenoidalis 102 verankert. Von besonderer Bedeutung ist hierbei, dass - wie insbesondere der Darstellung in Fig. 15 entnommen werden kann - die Grundplatte 8 in Richtung des Angulus Inferior versetzt an der Cavitas Glenoidalis 102 angeordnet ist. Hierdurch wird ein Anstoßen des Gelenkpfannenrands 106 bei zum Oberkörper hin abgewinkelten Arm weitestgehend vermieden. Selbst bei einem extrem starken Anwinkeln des Oberarms, wie es in Fig. 16 dargestellt ist, kann ein Anstoßen des Gelenkpfannenrands 106 an die Verankerungselemente nicht auftreten.

Diese angestrebte versetzte Anordnung der Grundplatte 8 wird hierbei insbesondere dadurch begünstigt, dass das Zentrum 47 der Kalottenform der Bodenfläche 22 der Grundplatte 8 exzentrisch zur Mittelachse 45 der Grundplatte 8 ausgebildet ist. Insbesondere ist dieses Zentrum 47 in entgegengesetzter Richtung gegenüber dem Angulus Inferior 90 der Scapula 88 verschoben. Diese besondere Ausgestaltung hat zur Folge, dass die Oberfläche der Cavitas Glenoidalis 102 eine für eine Montage der Schulterprothese 2 besonders bevorzugte kugelabschnittförmige Vertiefung aufweisen kann, da aufgrund der versetzten Anordnung das Zentrum des Auflagebereichs der Bodenfläche 22 nunmehr mit dem Zentrum 47 der Kalottenform zusammenfällt. Die vor der Montage der Schulterprothese 2 erforderliche Bearbeitung der Cavitas Glenoidalis 102 ist deshalb für einen Operateur verhältnismäßig einfach durchzuführen, wobei trotz allem ein gleichmäßiges Anliegen der Bodenfläche 22 an der Cavitas Glenoidalis 102 über den gesamten Kontaktflächenbereich hinweg erzielt werden kann. Wesentlich hierbei ist, dass die Form der Grundplatte 8 in optimaler Weise an die gegebenen Knochenverhältnisse angepasst ist, wodurch eine einfache aber auch besonders sichere Befestigung der Prothese 2 an dem Schulterblatt ermöglicht wird. Dies stellt einen entscheidenden Vorteil im Vergleich zu Lösungen aus dem Stand der Technik dar, bei denen beispielsweise die Bodenfläche der Grundplatte eben war und dementsprechend der Knochen stark bearbeitet bzw. manipuliert werden musste, um ein Befestigen der Prothese zu ermöglichen.

Die erfindungsgemäße Verwendung der beiden Vorsprünge 18 an der Grundplatte 8 ermöglicht es darüber hinaus auch, auf den Einsatz einer nach unten gerichteten Verankerungsschraube zu verzichten. Selbst für den Fall, dass der Arm - wie in Fig. 16 dargestellt - extrem stark angewinkelt wird und dementsprechend im Laufe der Zeit eine Abtragung des Knochenmaterials des Schulterblatts 88 an der entsprechenden Kontaktstelle auftritt, kann nicht der Fall auftreten, dass zu irgendeinem Zeitpunkt der Gelenkpfannenrand 106 mit den Vorsprüngen 18 und/oder einer der Verankerungsschrauben 26, 28, 30 kollidiert. Die Gefahr, dass im Laufe der Zeit eine der Schrauben 26, 28, 30 beschädigt wird und dementsprechend eine sichere Verankerung des Gelenkkopfs 6 am Schulterblatt nicht mehr sichergestellt ist, wird auf diese Weise vermieden.

Die Verwendung der beiden Vorsprünge 18 ermöglicht darüber hinaus auch die etwas zentralere Anordnung der beiden mittleren Verankerungsschrauben 28, 30. Da das Knochenmaterial des Schulterblatts an der Cavitas Glenoidalis 102 verhältnismäßig dünn ist, ist auf diese Weise trotz allem sichergestellt, dass die Schrauben 28, 30 sicher in das Knochenmaterial eingebracht werden und dieses über eine ausreichende Länge hinweg durchdringen können. Hierdurch wird die Verankerung des Gelenkkopfs 6 also nochmals zusätzlich verbessert.

Schließlich ermöglichen die beiden Vorsprünge 18 auch eine lagerichtige und verdrehsichere Anordnung der Grundplatte 18 an der zuvor bearbeiteten Cavitas Glenoidalis 102. Üblicherweise werden die entsprechenden Löcher für die Vorsprünge 18 vor dem endgültigen Montieren der Schulterprothese 2 mit Hilfe einer Schablone bzw. Positionierlehre markiert, vorgebohrt und dann in das Knochenmaterial eingebracht. Bei der Verwendung eines einzigen Vorsprungs 18 gemäß den bisherigen Lösungen konnte bei der anschließenden Montage der Grundplatte 8 ein Verdrehen dieser gegenüber der vorab definierten anterior/posteriore sowie inferior/superiore Rotationsausrichtung nicht ausgeschlossen werden, was letztendlich zu einer Fehlstellung bzw. falschen Ausrichtung der Schrauben und damit des vollständigen Gelenkkopfs 6 führte. Die Folge hiervon war, das die Verankerungsschrauben oftmals in Bereiche ragten, in denen kein Knochenmaterial vorhanden war. Diese Problematik besteht nunmehr bei dem erfindungsgemäßen Einsatz von zwei Vorsprüngen 18 nicht, da die beiden Öffnungen für die Vorsprünge eine Rotationsausrichtung für die Grundplatte 8 definieren, so dass mit einem verhältnismäßig geringen Aufwand eine exakte und sichere Befestigung der Schulterprothese am Schulterblatt ermöglicht ist.

Die Nutzung zweier Vorsprünge an der Grundplatte bringt also grundsätzlich Vorteile mit sich, auch für den Fall, dass die Bodenfläche nicht die oben beschriebene Exzentrität aufweist. Zwar führt diese besondere Ausgestaltung zu zusätzlichen Vorteilen, die Erfindung bezieht sich allerdings allgemein auf inverse Schulterprothesen, beispielsweise auch auf klassische Ausführungsformen, bei denen die Bodenfläche der Grundplatte eben ausgestaltet ist oder bei denen die Bodenfläche der Grundplatte kalottenförmig, aber ohne Exzentrizität des Kalottenmittelpunktes zur Mittelachse der Grundplatte ausgestaltet ist.

Eine alternative Ausführungsform für die Grundplatte 8 und den Gelenkkopf 6 ist beispielsweise in den Figuren 17 bis 19 dargestellt, wobei Elemente dieser beiden Teile, welche identisch zu den Teilen der zuvor beschriebenen Grundplatte 8 und dem Gelenkkopf 6 sind, mit den gleichen Bezugszeichen versehen sind. Nachfolgend sollen lediglich die Unterschiede dieser Variante gegenüber dem ersten Ausführungsbeispiel besprochen werden.

Der wesentlichen Unterschied dieser zweiten Variante besteht in der Ausgestaltung des Kontaktbereichs zwischen Grundplatte 8 und Gelenkkopf 6. Hierbei ist nun an der dem Gelenkkopf 6 zugewandten Seite der Grundplatte 8 eine Vertiefung bzw. kreisförmige Ausnehmung 118 vorgesehen, in welche ein entsprechender zylinder-artiger Vorsprung 116 an der Unterseite des Gelenkkopfs 6 eingreift. Die Aufnahme radialer Kräfte im Zusammengefügten Zustand wird durch diesen Eingriff nochmals verbessert.

Ein weiterer Unterschied besteht darin, dass sich eine zentrale Bohrung 120 durch den Gelenkkopf 6 erstreckt, welche in eine entsprechende Bohrung 122 in der Grundplatte 8 übergeht. Beide Bohrungen 120, 122 können nun zur Aufnahme eines - nicht dargestellten - Führungsbzw. Zentrierstifts verwendet werden, welcher die Anordnung des Gelenkkopfs 6 auf der Grundplatte 8 erleichtert.

Hinsichtlich der weiteren Ausgestaltungen stimmt die zweite Variante mit dem ersten Ausführungsbeispiel überein. Wiederum ist also die Bodenfläche 22 der Grundplatte 8 mit zwei Vorsprüngen 18 ausgebildet und mit einer exzentrisch ausgebildeten Kalottenform versehen. Wie bereits erwähnt wurde, könnte allerdings die Kalottenform auch zentrisch ausgebildet oder die Bodenfläche 22 der Grundplatte 8 sogar eben sein.

Insgesamt erlaubt also die erfindungsgemäße Lösung nicht nur eine einfach durchzuführende sondern auch eine besonders zuverlässige Montage des Gelenkkopfs am Schulterblatt. Ferner sind die einzelnen Komponenten der erfindungsgemäßen Schulterprothese derart ausgeführt, dass eine Beschädigung der zur Verankerung am Schulterblatt bestimmten Elemente, welche insbesondere durch das niemals vollständig zu vermeidende Inferior Notching hervorgerufen wird, vermieden werden kann.

## Patentansprüche

1. Schulterprothese (2) mit einer Gelenkpfanne (4) und einem mit dieser zusammenwirkenden Gelenkkopf (6), welcher mit einer scapulaseitigen Grundplatte (8) nach Art einer Klemmverbindung (44) zusammenwirkt,
wobei der Gelenkkopf (6) mit einer an diesem ausgebildeten Aufnahme (42) auf die Grundplatte (8) aufgesteckt ist, welche mit zwei an der Grundplatte (8) angeordneten, zapfenartigen Vorsprüngen (18) an der Cavitas Glenoidalis (102) der Scapula (8) verankerbar ist,
**dadurch gekennzeichnet,**
**dass** die Grundplatte (8) eine zur Anlage an der Cavitas Glenoidalis (102) der Scapula (88) vorgesehene kalottenförmige Bodenfläche (22) aufweist, wobei das Zentrum (47) der Kalottenform exzentrisch zur Mittelachse (45) der Grundplatte (8) angeordnet ist.

2. Schulterprothese nach Anspruch 1,
**dadurch gekennzeichnet,**
**dass** die Grundplatte (8) in Richtung auf einen Angulus Inferior (90) der Scapula (88) versetzt auf der Cavitas Glenoidalis (102) befestigbar ist.

3. Schulterprothese nach Anspruch 1 oder 2,
**dadurch gekennzeichnet,**
**dass** das Zentrum (47) der Kalottenform der Bodenfläche (22) auf einer Verbindungsgeraden zwischen den beiden Vorsprüngen (18) angeordnet ist.

4. Schulterprothese nach einem der vorherigen Ansprüche,
**dadurch gekennzeichnet,**
**dass** das Zentrum (47) der Kalottenform der Bodenfläche (22) in eine gegenüber dem Angulus Inferior (90) der Scapula (88) entgegengesetzte Richtung verschoben ist.

5. Schulterprothese nach einem der vorherigen Ansprüche,
**dadurch gekennzeichnet,**
**dass** die Grundplatte (8) zusätzlich mit Verankerungselementen (26, 28, 30), welche in an der Grundplatte (8) vorgesehenen Bohrungen (36, 38, 40) aufnehmbar sind, an der Cavitas Glenoidalis (102) der Scapula (88) verankerbar ist.

6. Schulterprothese nach Anspruch 5,
**dadurch gekennzeichnet,**
**dass** zumindest eine der Bohrungen (36, 38, 40) seitlich der Verbindungsgeraden zwischen den beiden Vorsprüngen (18) angeordnet ist.

7. Schulterprothese nach Anspruch 6,
**dadurch gekennzeichnet,**
**dass** zu beiden Seiten der Verbindungsgeraden zwischen den beiden Vorsprüngen (18) jeweils eine Bohrung (36, 38, 40) zur Aufnahme eines Verankerungselements (26, 28, 30) angeordnet ist.

8. Schulterprothese nach einem der Ansprüche 5 bis 7,
**dadurch gekennzeichnet,**
**dass** die Bohrungen (36, 38, 40) an einer der Scapula (88) abgewandten Seite der Grundplatte (8) Versenkungen (68) für an den Verankerungselementen (26, 28, 30) vorgesehene Köpfe aufweisen.

9. Schulterprothese nach einem der Ansprüche 5 bis 8,
**dadurch gekennzeichnet,**
**dass** die Bohrungen (36, 38, 40) an der Grundplatte (8) zur Führung der Verankerungselemente (26, 28, 30) derart ausgebildet sind, dass zumindest ein Verankerungselement (26, 28, 30) bei endmontierter Schulterprothese (2) seitlich weggespreizt ausgerichtet ist.

10. Schulterprothese nach Anspruch 9,
**dadurch gekennzeichnet,**
**dass** die weiteren Bohrungen (38, 40) an der Grundplatte (8) zur Führung der Verankerungselemente (28,30) derart ausgebildet sind, dass die zugehörigen Verankerungselemente (28, 30) mit einem geringfügigen Spielraum im wesentlichen parallel zu den Vorsprüngen (18) ausgerichtet sind.

11. Schulterprothese nach einem der Ansprüche 5 bis 10,
**dadurch gekennzeichnet,**
**dass** die Verankerungselemente (26, 28, 30) als Verankerungsschrauben ausgebildet sind.

12. Schulterprothese nach Anspruch 11,
**dadurch gekennzeichnet,**
**dass** an den Bohrungen (36, 38, 40) zur Aufnahme einer gespreizt ausgerichteten Verankerungsschraube ein Schraubgewinde (66) zur Ausbildung einer Schraubverbindung zwischen der Grundplatte (8) und der entsprechenden Verankerungsschraube vorgesehen ist.

13. Schulterprothese nach einem der vorherigen Ansprüche,
**dadurch gekennzeichnet,**
**dass** die Aufnahme (42) des Gelenkkopfs (6) zentrisch zu dessen Mittelachse (46) ausgebildet ist.

14. Schulterprothese nach einem der vorherigen Ansprüche,
**dadurch gekennzeichnet,**
**dass** eine Aufnahmeseitenfläche (52) am Gelenkkopf (6) und eine mit dieser zusammenwirkende Mantelfläche (50) der Grundplatte (8) zur Ausbildung der Klemmverbindung (44) kreisförmig ausgebildet sind.

15. Schulterprothese nach einem der vorherigen Ansprüche,
**dadurch gekennzeichnet,**
**dass** an einer dem Angulus Inferior (90) der Scapula (88) abgewandten Seite am Gelenkkopf (6) eine Nut (62) vorgesehen ist, mit welcher die Grundplatte (8) mit einer an dieser ausgebildeten Feder (60) zusammenwirkt.

16. Schulterprothese nach einem der Ansprüche 5 bis 12 und Anspruch 15,
**dadurch gekennzeichnet,**
**dass** im Bereich der Feder (60) an der Grundplatte (8) eine Bohrung (36) ausgebildet ist.

17. Schulterprothese nach einem der vorherigen Ansprüche,
**dadurch gekennzeichnet,**
**dass** die Vorsprünge (18) nach Art eines Dübels Rippen (24) aufweisen.

18. Schulterprothese nach einem der vorherigen Ansprüche,
**dadurch gekennzeichnet,**
**dass** die Vorsprünge (18) einstückig an der Bodenplatte (8) ausgebildet sind.

19. Schulterprothese nach einem der vorherigen Ansprüche,
**dadurch gekennzeichnet,**
**dass** in Verlängerung zu einer am Gelenkkopf (6) vorgesehenen Ausnehmung (86) an der Grundplatte (8) eine Bohrung (82) im Bereich eines der beiden Vorsprünge (18) ausgebildet ist, wobei sich ein Zentrierstift (87) sowohl in die Ausnehmung (86) als auch in die Bohrung (82) erstreckt.

20. Schulterprothese nach einem der vorherigen Ansprüche,
**dadurch gekennzeichnet,**
**dass** die Mantelfläche des Gelenkkopfs (6) an einer dem Angulus Inferior (90) zugewandten Seite in Art einer Lippe (70) verlängert ist.

## Claims

1. Shoulder prosthesis (2) with a joint socket (4) and a joint head (6) which cooperates with the latter and cooperates with a base plate (8) on the scapula side in the manner of a clamping connection (44),
wherein the joint head (6) is fitted by means of a mounting receptacle (42) formed in the latter onto the base plate (8) which can be anchored to the cavitas glenoidalis (102) of the scapula (8) with two peglike projections (18) arranged on the base plate (8),
**characterised in that**
the base plate (8) exhibits a calotte-shaped bottom face (22) which is designed to bear on the cavitas glenoidalis (102) of the scapula (88), wherein the centre (47) of the calotte shape is arranged eccentrically in relation to the central axis (45) of the base plate (8).

2. Shoulder prosthesis according to claim 1,
**characterised in that**
the base plate (8) can be fastened on the cavitas glenoidalis (102) so as to be offset in the direction of an angulus inferior (90) of the scapula (88).

3. Shoulder prosthesis according to claim 1 or 2,
**characterised in that**
the centre (47) of the calotte shape of the bottom face (22) is arranged on a connecting straight line between the two projections (18).

4. Shoulder prosthesis according to one of the preceding claims,
**characterised in that**
the centre (47) of the calotte shape of the bottom face (22) is displaced in an opposite direction to the angulus inferior (90) of the scapula (88).

5. Shoulder prosthesis according to one of the preceding claims,
**characterised in that**
the base plate (8) can also be anchored to the cavitas glenoidalis (102) of the scapula (88) by means of anchoring elements (26, 28, 30) which can be received in drillings (36, 38, 40) provided in the base plate (8).

6. Shoulder prosthesis according to claim 5,
**characterised in that**
at least one of the drillings (36, 38, 40) is arranged to the side of the connecting straight line between the two projections (18).

7. Shoulder prosthesis according to claim 6,
**characterised in that**
in each case a drilling (36, 38, 40) is arranged on both sides of the connecting straight line between the two projections (18) to receive an anchoring element (26, 28, 30).

8. Shoulder prosthesis according to one of claims 5 to 7,
**characterised in that**
on a side of the base plate (8) facing away from the scapula (88) the drillings (36, 38, 40) exhibit countersinking recesses (68) for heads provided on the anchoring elements (26, 28, 30).

9. Shoulder prosthesis according to one of claims 5 to 8,
**characterised in that**
the drillings (36, 38, 40) in the base plate (8) for guiding the anchoring elements (26, 28, 30) are designed in such a way that at least one anchoring element (26, 28, 30) is aligned spread away laterally when the shoulder prosthesis (2) is finally fitted in place.

10. Shoulder prosthesis according to claim 9,
**characterised in that**
the further drillings (38, 40) in the base plate (8) for guiding the anchoring elements (28, 30) are designed in such a way that the associated anchoring elements (28, 30) are aligned with a tiny amount of play essentially parallel to the projections (18).

11. Shoulder prosthesis according to one of claims 5 to 10,
**characterised in that**
the anchoring elements (26, 28, 30) are embodied in the form of anchoring screws.

12. Shoulder prosthesis according to claim 11,
**characterised in that**
a screw thread (66) is provided in the drillings (36, 38, 40) for receiving an anchoring screw aligned spread to form a screwed connection between the base plate (8) and the corresponding anchoring screw.

13. Shoulder prosthesis according to one of the preceding claims,
**characterised in that**
the mounting receptacle (42) of the joint head (6) is formed centrally in relation to its central axis (46).

14. Shoulder prosthesis according to one of the preceding claims,
**characterised in that**
a mounting receptacle side face (52) on the joint head (6) and an external face (50) of the base plate (8) cooperating with this mounting receptacle side face are embodied with a circular shape to form the clamping connection (44).

15. Shoulder prosthesis according to one of the preceding claims,
**characterised in that**
provided in the joint head (6) on a side facing away from the angulus inferior (90) of the scapula (88) there is a groove (62) with which the base plate (8) cooperates by means of a tongue (60) formed on the latter.

16. Shoulder prosthesis according to one of claims 5 to 12 and claim 15,
**characterised in that**
a drilling (36) is formed in the region of the tongue (60) on the base plate (8).

17. Shoulder prosthesis according to one of the preceding claims,
**characterised in that**
the projections (18) exhibit ribs (24) in the manner of a dowel.

18. Shoulder prosthesis according to one of the preceding claims,
**characterised in that**
the projections (18) are formed in one piece on the base plate (8).

19. Shoulder prosthesis according to one of the preceding claims,
**characterised in that**
a drilling (82) is formed in the base plate (8) in the region of one of the two projections (18) in an extension of an opening (86) provided in the joint head (6), wherein a centering pin (87) extends both into the opening (86) and into the drilling (82).

20. Shoulder prosthesis according to one of the preceding claims,
**characterised in that**
the external face of the joint head (6) on a side facing towards the angulus inferior (90) is elongated in the manner of a lip (70).

## Revendications

1. Prothèse d'épaule (2) comprenant un cotyle (4) et une tête d'articulation (6) coopérant avec ledit cotyle, laquelle coopère avec une plaque de base (8) côté omoplate à la manière d'un assemblage par serrage (44),
sachant que la tête d'articulation (6) est emboîtée sur la plaque de base (8) par un logement (42) réalisé au niveau de ladite tête d'articulation, lequel logement peut être ancré au niveau de la cavité glénoïde (102) de l'omoplate (8) par deux parties faisant saillie (18) de type tenon disposées au niveau de la plaque de base (8),
**caractérisée en ce**
**que** la plaque de base (8) présente une surface de fond (22) en forme de calotte prévue pour venir en appui au niveau de la cavité glénoïde (102) de l'omoplate (88), sachant que le centre (47) de la forme de calotte est disposé de manière excentrique par rapport à l'axe central (45) de la plaque de base (8).

2. Prothèse d'épaule selon la revendication 1,
**caractérisée en ce**
**que** la plaque de base (8) peut être fixée sur la cavité glénoïde (102) de manière décalée en direction d'un angle inférieur (90) de l'omoplate (88).

3. Prothèse d'épaule selon la revendication 1 ou 2,
**caractérisée en ce**
**que** le centre (47) de la forme de calotte de la surface de fond (22) est disposé sur une droite d'assemblage entre les deux parties faisant saillie (18).

4. Prothèse d'épaule selon l'une quelconque des revendications précédentes,
**caractérisée en ce**
**que** le centre (47) de la forme de calotte de la surface de fond (22) est glissé dans une direction opposée par rapport à l'angle inférieur (90) de l'omoplate (88).

5. Prothèse d'épaule selon l'une quelconque des revendications précédentes,
**caractérisée en ce**
**que** la plaque de base (8) peut être ancrée au niveau de la cavité glénoïde (102) de l'omoplate (88) en supplément par des éléments d'ancrage (26, 28, 30), qui peuvent être logés dans des alésages (36, 38, 40) prévus au niveau de la plaque de base (8).

6. Prothèse d'épaule selon la revendication 5,
**caractérisée en ce**
**qu'**au moins un des alésages (36, 38, 40) est disposé sur le côté de la droite d'assemblage entre les deux parties faisant saillie (18).

7. Prothèse d'épaule selon la revendication 6,
**caractérisée en ce**
**que** respectivement un alésage (36, 38, 40) servant à recevoir un élément d'ancrage (26, 28, 30) est disposé de part et d'autre de la droite d'assemblage entre les deux parties faisant saillie (18).

8. Prothèse d'épaule selon l'une quelconque des revendications 5 à 7,
**caractérisée en ce**
**que** les alésages (36, 38, 40) présentent, au niveau d'un côté, opposé à l'omoplate (88), de la plaque de base (8), des noyures (68) destinées à des têtes prévues au niveau des éléments d'ancrage (26, 28, 30).

9. Prothèse d'épaule selon l'une quelconque des revendications 5 à 8,
**caractérisée en ce**
**que** les alésages (36, 38, 40) sont réalisés au niveau de la plaque de base (8) aux fins du guidage des éléments d'ancrage (26, 28, 30) de telle manière qu'au moins un élément d'ancrage (26, 28, 30) est orienté de manière à s'écarter sur le côté lorsque le montage de la prothèse d'épaule (2) est finalisé.

10. Prothèse d'épaule selon la revendication 9,
**caractérisée en ce**
**que** les autres alésages (38, 40) sont réalisés au niveau de la plaque de base (8) aux fins du guidage des éléments d'ancrage (28, 30) de telle manière que les éléments d'ancrage (28, 30) associés sont orientés essentiellement de manière parallèle par rapport aux parties faisant saillie (18) avec un léger jeu.

11. Prothèse d'épaule selon l'une quelconque des revendications 5 à 10,
**caractérisée en ce**
**que** les éléments d'ancrage (26, 28, 30) sont réalisés sous la forme de vis d'ancrage.

12. Prothèse d'épaule selon la revendication 11,
**caractérisée en ce**
**qu'**un filetage de vis (66) servant à réaliser un assemblage par vissage entre la plaque de base (8) et la vis d'ancrage correspondante est prévu au niveau des alésages (36, 38, 40) servant à recevoir une vis d'ancrage orientée de manière écartée.

13. Prothèse d'épaule selon l'une quelconque des revendications précédentes,
**caractérisée en ce**
**que** le logement (42) de la tête d'articulation (6) est réalisé de manière centrée par rapport à l'axe central (46) de cette dernière.

14. Prothèse d'épaule selon l'une quelconque des revendications précédentes,
**caractérisée en ce**
**qu'**une surface latérale de logement (52) sur la tête d'articulation (6) et une surface enveloppante (50) coopérant avec cette dernière, de la plaque de base (8) sont réalisées de manière à présenter une forme circulaire afin de réaliser l'assemblage par serrage (44).

15. Prothèse d'épaule selon l'une quelconque des revendications précédentes,
**caractérisée en ce**
**qu'**est prévue, au niveau d'un côté, opposé à l'angle inférieur (90) de l'omoplate (88), au niveau de la tête d'articulation (6), une rainure (62), avec laquelle la plaque de base (8) coopère par un ressort (60) réalisé au niveau de ladite plaque de base.

16. Prothèse d'épaule selon l'une quelconque des revendications 5 à 12 et selon la revendication 15,
**caractérisée en ce**
**qu'**un alésage (36) est réalisé dans la zone du ressort (60) au niveau de la plaque de base (8).

17. Prothèse d'épaule selon l'une quelconque des revendications précédentes,
**caractérisée en ce**
**que** les parties faisant saillie (18) présentent, à la manière d'une cheville, des nervures (24).

18. Prothèse d'épaule selon l'une quelconque des revendications précédentes,
**caractérisée en ce**
**que** les parties faisant saillie (18) sont réalisées d'un seul tenant au niveau de la plaque de fond (8).

19. Prothèse d'épaule selon l'une quelconque des revendications précédentes,
**caractérisée en ce**
**qu'**un alésage (82) dans la zone d'une des deux parties faisant saillie (18) est réalisé dans le prolongement par rapport à un évidement (86) prévu au niveau de la tête d'articulation (6) au niveau de la plaque de base (8), sachant qu'une tige de centrage (87) s'étend aussi bien dans l'évidement (86) que dans l'alésage (82).

20. Prothèse d'épaule selon l'une quelconque des revendications précédentes,
**caractérisée en ce**
**que** la surface enveloppante de la tête d'articulation (6) est prolongée au niveau d'un côté tourné vers l'angle inférieur (90) à la manière d'une lèvre (70).
